# EUROPEAN PATENT APPLICATION

(11) **EP 4 717 076 A1**
(43) Date of publication of application: **01.04.2026**
(21) Application number: 24809955.8
(22) Date of filing: 29.01.2024
(51) Int. Cl.: A01G 9/18, A01G 22/30, C12M 1/04, B01D 53/84

(54) **METHOD AND SYSTEM FOR COLLECTING AND FIXING CARBON DIOXIDE BASED ON CARBON SEQUESTRATION PLANT PEAT MOSS, AND USE**

(30) Priority: 23.05.2023 CN 202310582826
(71) Applicant: East China Normal University, Putuo District, Shanghai 200061 (CN)
(72) Inventor: SUN, Yue, Shanghai 200241 (CN); XUE, Yongjun, Shanghai 200241 (CN)
(74) Representative: Murgitroyd & Company
(86) International application number: PCT/CN2024/074385
(87) International publication number: WO 2024/239705

(57) **Abstract**

Provided is a method for collecting and fixing carbon dioxide based on carbon sequestration plant peat moss, which comprises the following steps: planting peat moss in a container having good sealing performance; and mixed gas containing high-concentration carbon dioxide entering the culture container from an air inlet for collecting and fixing carbon dioxide. The method can stably collect and fix and sequestrate carbon dioxide in the air with a low energy consumption. Also provided are a system for collecting and fixing carbon dioxide based on carbon sequestration plant peat moss and use of the system for collecting and fixing carbon dioxide in planting peat moss and collecting methane.

## Description

### Technical Field

The present invention relates to the field of peat moss, and specifically relates to a method and system for collecting and fixing carbon dioxide based on carbon sequestration plant peat moss, and use.

### Background of the Invention

The consumption of massive quantities of fossil fuels in human industrial and domestic activities, whether through direct combustion or processing into industrial products, releases substantial amounts of carbon dioxide. Moreover, processes involving microbial fermentation or the treatment of organic waste also release a significant amount of carbon dioxide. Now, the world is under rapid temperature rise, and reducing atmospheric carbon dioxide levels is an urgent task for all mankind. One approach to emission reduction is to collect, fix, or sequester carbon dioxide before it is discharged into the atmosphere. Existing methods for collecting or fixing carbon dioxide include physical and chemical means, as well as carbon dioxide capture using carbonic anhydrase derived from microorganisms. However, these methods have significant drawbacks, such as high energy costs, environmental risks associated with the use of chemical compounds, and unstable fixation stability. Some methods for collecting carbon dioxide are limited to collecting carbon dioxide dissolved in water. Due to the limited solubility of carbon dioxide in water, it is difficult to improve the efficiency of such methods.

Photosynthesis, which utilizes light energy to fix carbon dioxide and synthesize sugars, represents the most efficient and energy-saving chemical reaction for carbon dioxide capture in the world. Concurrently, a series of biochemical reactions occur within the plant, ultimately fixing carbon dioxide into recalcitrant plant cell wall components, such as lignin. However, spermatophytes (e.g., trees) are voluminous and require substantial planting space, making them difficult to cultivate in the form of a bioreactor. Other micro-plants, such as algae, can capture carbon dioxide during growth, but they are incapable of synthesizing lignin and thus cannot permanently fix carbon dioxide. The simplest plants capable of synthesizing lignin are mosses. Peat moss is a large perennial moss. Due to the inherent physiological characteristics of peat moss, the biomass formed through carbon sequestration is resistant to degradation and can be fixed for the long term. Therefore, peat moss is also referred to as carbon sequestration plant. Provided uses peat moss as the primary biological agent for absorbing and fixing carbon dioxide. By configuring rational air inlet, outlet, and detection systems, and providing the necessary conditions for the growth of peat moss, a bioreactor is constructed for the efficient fixation and sequestration of carbon dioxide.

Currently, facility agriculture and soil improvement require significant quantities of artificial substrates, of which peat is an essential and primary component. Peat is classified as a non-metallic strategic resource, and its extraction is prohibited in China. Research results in recent years indicate that dried peat moss has physicochemical properties identical to those of peat and can serve as a substitute for peat as the primary component of artificial substrates. Dried peat moss is increasingly being incorporated into artificial substrate formulations. In particular, the planting of orchids requires dried peat moss as a substrate. Thus, the market demand for dried peat moss, as the optimal replacement for peat, is steadily on the rise.

### Summary of the Invention

In order to overcome the aforementioned drawbacks, the present invention provides an efficient carbon-negative technology, which is applicable to scenarios requiring the reduction of carbon dioxide concentration prior to the emission of gas mixture containing high concentrations of carbon dioxide generated from industrial, agricultural, or livestock production, as well as various waste treatment processes. Such scenario requires the prior removal of components within the gas mixture that may be harmful to plant growth, such as ammonia, aromatic hydrocarbons, SO₂, NO, CO, and dust particles. By utilizing this carbon-negative technology, the carbon dioxide concentration in the gas mixture can be reduced to levels below the atmospheric average, while the oxygen concentration will rise. The present invention relates to the long-term stable collection and fixation of carbon dioxide from waste gas on a large scale, such as carbon dioxide in flue gas emitted from coal-fired or NG-fired factory boilers, carbon dioxide in waste gas released from industrial fermentation, carbon dioxide released during the treatment of wet waste, carbon dioxide released during the industrial processing of carbonates, and carbon dioxide released from livestock farms. The present invention involves the carbon sequestration plant peat moss and other plants with similar functions.

The carbon-negative technology described herein comprises a system for fixing carbon dioxide, which is composed of three parts: an air inlet control device, a plant culture device, and an air outlet control device. These devices are interconnected. The carbon dioxide concentration within the devices is monitored by real-time high-precision detectors. The direction of gas flow is controlled by gas circulation pumps and vent valves. This system is applicable to the carbon dioxide fixation of gas mixture containing relatively high-concentrations carbon dioxide from various sources. As the gas mixture containing relatively high-concentration carbon dioxide remains in the system for a period of time, the carbon dioxide concentration in the gas mixture will decrease to below the atmospheric average level. Green plants grow rapidly within this system. Currently, the carbon sequestration plant peat moss is applied in this system. However, other small or micro plants with high carbon fixation efficiency or strong market demand may also be planted depending on the environmental requirements of specific scenarios.

The present invention provides use of the high-efficiency carbon sequestration plant peat moss in the field of carbon sequestration. When peat moss grows in the open atmosphere, it does not exhibit a particularly significant capability for carbon dioxide fixation. However, under high carbon dioxide conditions, peat moss exhibits high photosynthetic efficiency. Furthermore, peat moss can tolerate relatively high oxygen concentrations and continuously fix carbon dioxide. Therefore, peat moss can be used to convert gas mixture containing high-concentration carbon dioxide into gas mixture containing low-concentration carbon dioxide and relatively high-concentration oxygen.

The present invention further provides a method for collecting methane with plants. Since methane-oxidizing bacteria can oxidize methane into carbon dioxide, and in natural peat moss wetlands, methane-oxidizing bacteria can exist in symbiosis on peat moss, the system can be used for collecting methane when the cultured peat moss is inoculated with methane-oxidizing bacteria.

The present invention further provides a method for planting peat moss under facility conditions.

The present invention further provides a set of bioreactors for producing peat moss.

The present invention further provides a method for fixing atmospheric carbon dioxide with plants. The present invention utilizes the carbon sequestration plant peat moss for carbon fixation. By integrating a device that controls air inlet and outlet and is coupled with the culture device, in conjunction with artificial planting method, peat moss can be successfully planted in environments with relatively high carbon dioxide concentrations. The fixation of atmospheric carbon dioxide can be achieved by planting peat moss. Peat moss can tolerate high-concentration carbon dioxide in the air and perform photosynthesis in such environments. Dried peat moss can substitute for peat when used as a substrate. Peat moss is an essential and effective component of artificial substrates in certain horticultural fields. Currently, peat moss is in high demand in the market. The peat moss planted by carbon dioxide fixation can be directly buried in the soil to increase organic carbon content in soil. It is also suitable for use as a horticultural substrate, effectively addressing the supply gap.

The present invention provides a method for collecting and fixing carbon dioxide based on carbon sequestration plant peat moss, comprising the following steps:
(1) planting peat moss in a container having good sealing performance;
(2) mixed gas containing high-concentration carbon dioxide entering the culture container from an air inlet for collecting and fixing carbon dioxide .

Regarding Step (1), the taxonomic group to which the peat moss belongs is the Phylum Bryophyta, including all wild plants of the genus peat moss, as well as related artificially bred varieties. Preferably, the species is Sphagnum Palustre and its varieties.

Regarding Step (1), the source of the peat moss is artificial planting. Preferably, the material is derived from tissue culture. The peat moss seedlings are derived from open-field artificial planting or indoor planting.

The facility planting includes simple plastic greenhouses, permanent steel-frame greenhouses, brick-and-wood structures, air-raid shelters, and other artificial planting environments capable of a certain degree of environmental regulation.

Regarding Step (1), the peat moss includes various growth stages of the moss, primarily protonemata and gametophytes; The gametophyte state is preferred.

Regarding Step (1), a dedicated lighting system is provided above the container for planting peat moss. The lighting system can provide a visible light source with a wavelength of 300-760nm required for plant growth, or an LED light source suitable for the growth of peat moss. A visible light source of 350-730 nm is preferred.

Regarding Step (1), the container is highly light-transmissive, with at least a top side thereof being light-transmissive, exhibiting high transmittance for visible light in the wavelength range of 350-750nm. Preferably, all sides of the container are light-transmissive.

The lighting system comprises a device for adjusting light intensity and photoperiod.

The light intensity for planting peat moss is 1,000-10,000lux; preferably 3,000-7,000lux.

The photoperiod set for peat moss is 8-14 hours of light per day; preferably 10-12 hours of light per day. Regarding Step (1), the container contains a certain water-absorbing and water-retaining substrate, which may be peat, dried peat moss, or other substrate components with moisture retention capabilities, or a mixture of two or more such components. It may be peat or a composite substrate containing peat, a composite substrate containing polyglutamic acid; or a composite culture medium containing agar. Preferably, the substrate is dried peat moss.

Regarding Step (1), the container may also contain sufficient aqueous solution for planting peat moss.

Regarding Step (1), the container is equipped with temperature and humidity sensors, which can transmit the humidity data within the container to an information terminal via wireless or wired means.

Regarding Step (1), the container features a sealable opening for feeding water and nutrients.

Regarding Step (1), when the humidity within the container falls below 80%, appropriate moisture must be replenished through the feeder to raise the internal humidity to above 90%, and preferably above 95%.

The planting humidity must be controlled at 75%-100%, preferably 85%-95%.

Regarding Step (1), the temperature for peat moss planting must be controlled at 15-28°C, preferably 18-22°C.

Regarding Step (1), mineral elements must be replenished periodically. Major nutrient elements include nitrate nitrogen, phosphorus, potassium, magnesium, iron, calcium, etc. Commercial compound nutrients may be used, or they may be prepared manually by the user.

In the mineral supplement mixture for peat moss, the concentration of nitrate nitrogen is 20mM or below, preferably 10mM or below.

In the mineral supplement mixture for peat moss, the concentration of phosphorus is 5mM or below, preferably 2mM or below.

In the mineral supplement mixture for peat moss, the concentration of potassium is 25mM or below, preferably 15mM or below.

In the mineral supplement mixture for peat moss, the concentration of magnesium is 5mM or below, preferably 2mM or below.

In the mineral supplement mixture for peat moss, the concentration of calcium is 10mM or below, preferably 5mM or below.

In the mineral supplement mixture for peat moss, the concentration of iron is 0.5mM or below, preferably 0.1mM or below.

Feed moderate amount of nutrients, and avoid excessive feeding. Preferably, compound nutrients are replenished when the plant growth slows down.

Regarding Step (1), the plant culture device is accessible, and can be harvested when the peat moss grows to a certain extent. New seedlings can be replaced.

Regarding Step (1), the container features an air inlet and/or an air outlet. Preferably, they are located at different positions.

The air inlet in Step (1) may feature a connecting tube in the container, with its opening preferably positioned near the plants.

The air outlet in Step (1) may feature a connecting tube in the container, with its opening preferably positioned away from the plants and the substrate/solution.

The container is equipped with ports connecting to the outside, including an air inlet and an air outlet. Regarding Step (2), preferably, the carbon dioxide concentration in the gas mixture is lower than 20,000ppm.

Regarding Step (2), if the concentration is higher than 20,000ppm, the gas must be diluted with air, N₂, or other gases that do not affect peat moss growth before entering the culture container, until the concentration drops to 20,000ppm.

Regarding Step (2), the gas mixture may be generated through various pathways. However, prior to entering the culture container, it must be purified to remove substances potentially toxic to the peat moss, such as ammonia, xylene, and formaldehyde.

The carbon dioxide exists in a gaseous state in a gas mixture. The carbon dioxide in the gas mixture may be produced by combustion. The carbon dioxide in the gas mixture may be produced by biological metabolism. The carbon dioxide in the gas mixture may be produced by the oxidation of CH₄ via biological or abiotic ways. The gas mixture may be diluted with N₂ before being introduced into the device for fixing carbon dioxide. The gas mixture contains no substances potentially toxic to the peat moss, such as ammonia, xylene, and formaldehyde.

The plant culture device is accessible, and can be harvested when the peat moss grows to a certain extent.

The present invention further provides a system for collecting and fixing carbon dioxide,comprising an air inlet control device, a plant culture device, and an air outlet control device, wherein:
The plant culture device is connected to the air inlet control device via an air inlet;
The air inlet control device is equipped with an air inlet control port which controls the entry of external air into the air inlet control device, as well as a vent connecting with the plant culture device;
The vent of the air inlet control device is equipped with a valve that controls the entry of gas into the plant culture device;
The plant culture device is in connection with the air outlet control device;
The air outlet control device is equipped with an exhaust control valve; the exhaust control valve is designed for manual or automatic control; wherein the automatically controlled exhaust control valve detects the carbon dioxide concentration within the air outlet control device and controls the opening of the exhaust control valve by determining whether the gas meets the discharge standard;
Where the exhaust control valve does not connect with the outside for venting, the exhaust control valve is connected to the air inlet control device;
The system for collecting and fixing carbon dioxide features a gas circulation pump designed to drive gas flow into the plant culture device; the gas circulation pump is installed at a position that can drive gas flow within the culture container.

The air inlet control port is equipped with a first vent valve;
The gas circulation pump is positioned between the air inlet control device and the plant culture device;
The air inlet control device comprises a first real-time carbon dioxide concentration sensor which is configured to transmit carbon dioxide concentration data of the air inlet control device to an information terminal via wired or wireless means;
The air outlet control device comprises a second carbon dioxide concentration detector which is configured to transmit carbon dioxide concentration data of the air outlet control device to an information terminal via wired or wireless means;
The plant culture device is provided with a lid at the top, which is provided with a nutrient feeder;
The gas outlet of the plant culture device is provided with a second vent valve;
The system for collecting and fixing carbon dioxide also comprises a visible light source.

The gas circulation pump is designed to drive the flow of gas into and within the plant culture device. The pump may be installed at any location capable of facilitating gas movement within the plant culture device. Preferably, it is positioned at the connection between the air inlet control device and the plant culture device.

The real-time carbon dioxide concentration detector may transmit carbon dioxide concentration data within the plant culture device to an information terminal via wired or wireless means. These detectors may be installed at any point within the gas circulation loop of the entire device. Preferably, they are installed within the air inlet control device. Upon startup of the system for collecting and fixing carbon dioxide herein, the operation proceeds as follows: First, the gas circulation pump is activated. Subsequently, the valve on the air outlet control device venting to the environment is opened, followed by the opening of the inlet valve on the air inlet control device. The conduit connecting the air inlet control device to the plant culture device must remain unobstructed. After a period of operation, the ambient air initially within the system is displaced by the gas mixture containing high-concentration carbon dioxide.

The vent valves on the plant culture device and the air outlet control device that vent to the environment are closed. The valve connecting the air outlet control device back to the air inlet control device is opened. The inlet control valve for admitting the external high-concentration carbon dioxide gas mixture is closed.

Thereafter, the gas mixture containing high-concentrations carbon dioxide circulates within the plant culture device and the air outlet control device.

When the carbon dioxide concentration within the system for collecting and fixing decreases to ambient levels, the valve on the air outlet control device venting to the environment is opened. The inlet valve on the air inlet control device is also opened. The conduit connecting the air inlet control device to the peat moss planting container must remain unobstructed. After a period of operation, the ambient air initially within the system is displaced by the gas mixture containing high-concentration carbon dioxide.

By repeating this cycle, continuous photosynthetic fixation of carbon dioxide is achieved.

When the peat moss reaches maturity, it can be harvested. New seedlings are then planted to continue the process of carbon dioxide collection, fixation, and sequestration.

Furthermore, by inoculating the peat moss with methane-oxidizing bacteria, the system can be extended to a system for collecting methane. Consequently, the present invention is also suitable for collecting methane.

The present invention further provides use of the system for collecting and fixing carbon dioxide in planting peat moss.

The present invention also provides use of the system for collecting and fixing carbon dioxide in collecting methane.

Beneficial effects of the present invention include: The present invention can stably collect, fix, and sequester atmospheric carbon dioxide under conditions of low energy consumption. This method is very efficient, requiring energy only for LED lighting and the peristaltic pump used for gas circulation. It involves minimal process steps and requires no specialized physical or chemical reaction apparatuses. Since plants are used in the collecting process, it is free from environmental risks. The plants harvested can be processed into marketable products, such as raw materials for substrate, offering good profit margins and further offsetting the costs of carbon dioxide collection. In summary, it represents a currently scarce carbon-negative technology. Implementing this method on a significant scale can fundamentally reduce carbon emissions in production systems and facilitate the realization of carbon neutrality.

### Brief Description of the Drawings

Fig. 1 is a schematic diagram of the system for collecting and fixing carbon dioxide. 1 - Air inlet control device; 2 - Plant culture device; 3 - Air outlet control device; 4 - First vent valve; 5 - Second vent valve; 6 - Exhaust control valve; 7 - First real-time carbon dioxide concentration sensor; 8 - Second carbon dioxide concentration detector; 9 - Gas circulation pump; 10 - Nutrient feeder; 11 - Lid; 12 - Visible light source.
Fig. 2 illustrates the changes in CO₂ concentration over time within the device for planting peat moss for the control group (without peat moss) in Example 1.
Fig. 3 illustrates the changes in CO₂ concentration over time within the device for planting peat moss for the experimental group (with peat moss planting) in Example 1.
Fig. 4 illustrates the changes in CO₂ concentration over time within the device for planting peat moss for experimental group (with peat moss planting) in Example 2.
Fig. 5 illustrates the changes in CO₂ concentration over time within the device for planting peat moss for the experimental group (with peat moss planting) in Example 3.

### Detailed Description of the Embodiments

The present invention will be described in further detail below in conjunction with the following specific examples and the accompanying drawings. Unless otherwise specified herein, the processes, conditions, and experimental methods used in the implementation of the present invention are consistent with common general knowledge in the art and are not subject to special restrictions by the present invention.

### Example 1

The air inlet of the plant culture device (containing peat moss) was connected to the vent of the air outlet control device. Approximately 0.2g of dry ice was added to the air inlet control device, and the system was sealed. Once the dry ice had completely sublimated, the carbon dioxide concentration detector indicated that the concentration had reached approximately 7,000ppm. The test was conducted at room temperature of 20°C, light intensity of 2,000lux, and internal humidity of 99%. Approximately 300cm² of healthy peat moss was added into the experimental group. The control group contains no plants; however, 200ml of water was added to maintain equivalent humidity levels. The CO₂ concentration in the system was measured hourly and plotted as a curve.

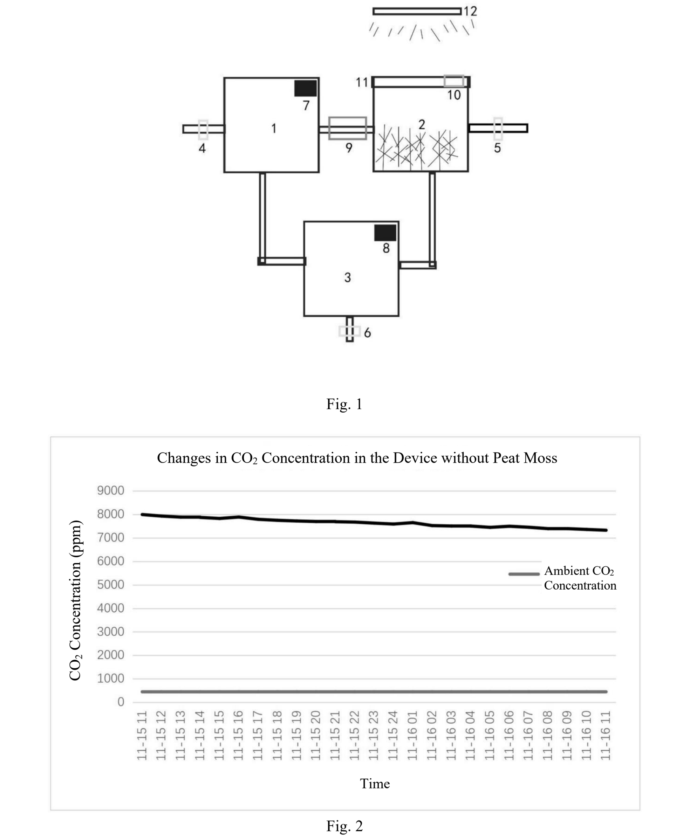

It can be observed that in the control group (see Fig. 2), the CO₂ concentration remained above 7,000ppm from 11:00 on November 15 to 11:00 on November 16. A slight decline in CO₂ concentration was observed, which is presumed to be caused by the partial dissolution of CO₂ into the water.

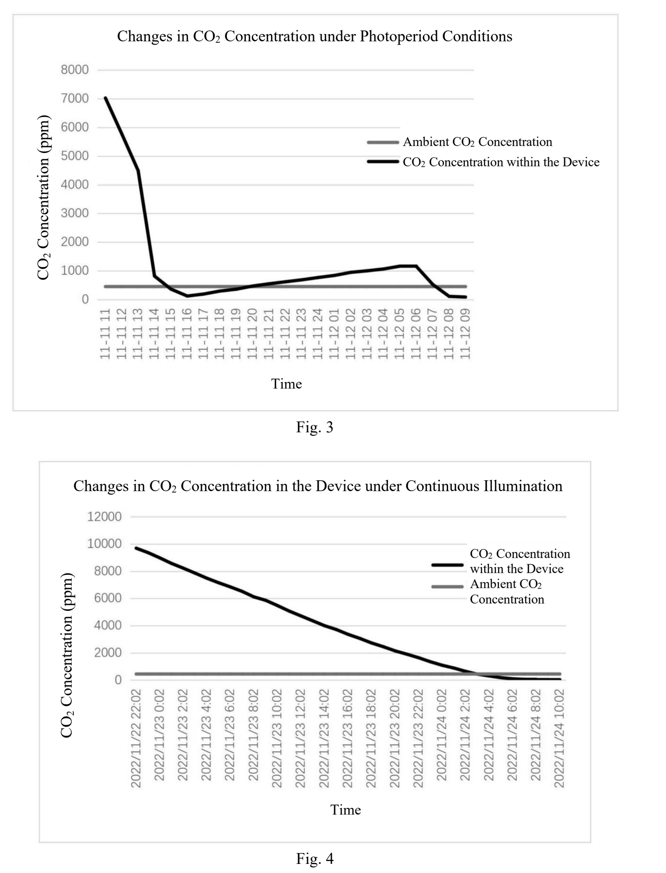

In the experimental group subjected to a photoperiod (see Fig. 3), the light period was from 06:00 to 16:00. During this time, the CO₂ concentration within the device decreased continuously, reaching a minimum of 112ppm. Such concentration is significantly lower than the ambient CO₂ concentration (450ppm). The dark period lasted from 16:00 to 06:00 on the following day. During this phase, the CO₂ concentration within the plant culture device rose continuously and slowly, peaking at 1,160ppm. Upon the resumption of illumination at 06:00 on the following day, the CO₂ concentration decreased rapidly again, dropping to 86ppm by 09:00. Since the CO₂ concentration within the plant culture device differed from the ambient CO₂ concentration for the vast majority of the 24-hour period, this demonstrates that the system is well airtight, without the possibility of leakage. As calculated with an initial concentration of 7,000ppm and an estimated volume of 15L, the device achieved the fixation of 0.105L of carbon dioxide within 24 hours.

### Example 2

The air inlet of the plant culture device (containing peat moss) was connected to the vent of the air outlet control device. Approximately 0.3g of dry ice was added to the air inlet control device, and the system was sealed. Once the dry ice had completely sublimated, the carbon dioxide concentration detector indicated that the concentration had reached approximately 9,700ppm. The test was conducted at room temperature of 20°C and internal humidity of 99%. Approximately 300cm² of healthy peat moss was added to the experimental group. The control group conditions were identical to those described in Example 1 of the present invention; therefore, the description is not repeated here.

Under continuous illumination from 22:00 to 10:00 on the following day, the CO₂ concentration within the device decreased continuously, dropping to as low as 29ppm (see Fig. 4). Such concentration is significantly lower than the ambient CO₂ concentration (450ppm). This demonstrates that the system is well airtight, thereby without the possibility of leakage. Consequently, the device achieved the fixation of approximately 0.1355L of carbon dioxide within 12 hours.

### Example 3

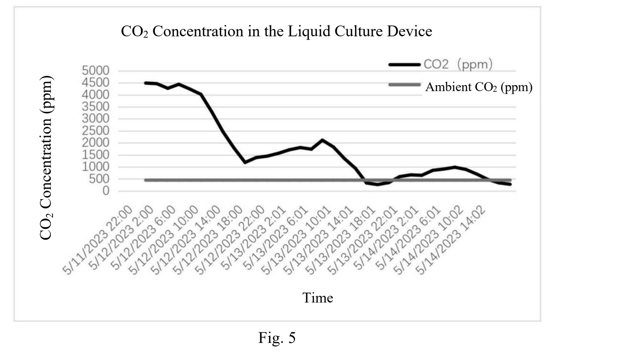

An aqueous solution containing compounded nutrients was added to the plant culture device of the present invention (wherein the plant is peat moss), and the peat moss was added to the aqueous solution. A tube connected to the air inlet of the plant culture device was submerged in the culture solution, while the opening of the exhaust pipe was above and away from the surface of the solution. The air inlet of the air inlet control device of the present invention was connected to the vent of the culture container. 500ml of nutrient solution and approximately 3g (fresh weight) of tissue-cultured peat moss were added to the plant culture container. One commercially available carbon dioxide-generating effervescent tablet and 500ml of water were added to the air inlet control device, and the system was sealed. The test was conducted at room temperature of 20°C. The CO₂ concentration in the system was measured hourly and plotted as a curve. Upon the reaction of the effervescent tablet with water and the complete release of carbon dioxide, the carbon dioxide concentration detector indicated that the concentration had reached approximately 4,000ppm. Day 1, 22:00 - Day 2, 08:00: A dark state without illumination was maintained. The CO₂ concentration in the system remained substantially unchanged. Day 2, 08:00 - 16:00: Illumination of 2,000lux was provided. It was observed that the system's CO₂ concentration decreased at a uniform rate, dropping to approximately 1,250ppm by 16:00. Day 2, 16:00 - Day 3, 08:00: The system operated under dark conditions, and the internal CO₂ concentration slowly rose to approximately 1,800ppm. Day 3, 08:00 - 16:00: Illumination of 2,000lux was provided. It was observed that the system's CO₂ concentration decreased at a uniform rate, dropping to approximately 400ppm by 16:00. Day 3, 16:00 - Day 4, 08:00: The system operated under dark conditions, and the internal CO₂ concentration slowly rose to approximately 1,000ppm. Day 4, 08:00 - 16:00: Illumination of 2,000lux was provided. It was observed that the system's CO₂ concentration decreased at a uniform rate, dropping to approximately 300ppm by 16:00. (see Fig. 5) Such concentration is significantly lower than the ambient CO₂ concentration (450ppm). This demonstrates that the system is well airtight, thereby without the possibility of leakage. The total gas volume of the device is approximately 1,200ml. Therefore, the 3g (fresh weight) of peat moss in the device achieved the following fixation results: approximately 3.3ml of CO₂ fixed during the 8 hours of illumination on the second day; 1.68ml of CO₂ fixed on the third day; and 0.84ml of CO₂ fixed on the fourth day. It is evident that the higher the concentration of introduced CO₂, the higher the CO₂ fixation efficiency.

The scope of protection of the present invention is not limited to the Examples described above. Any variations and advantages that can be conceived by those skilled in the art without departing from the spirit and scope of the inventive concept are encompassed within the present invention, and the scope of protection is defined by the appended Claims.

## Claims

1. A method for collecting and fixing carbon dioxide based on carbon sequestration plant peat moss, **characterized in that** it comprises the following steps:
(1) planting peat moss in a container having good sealing performance; and
(2) mixed gas containing high-concentration carbon dioxide entering the culture container from an air inlet for collecting and fixing carbon dioxide.

2. The method according to Claim 1, **characterized in that**:
The taxonomic group to which the peat moss belongs is the Phylum Bryophyta, including all wild plants of the genus peat moss, as well as related artificially bred varieties;
The source of the peat moss is artificial planting;
The peat moss includes various growth stages of the moss, including protonemata and gametophytes;
The peat moss seedlings are derived from open-field artificial planting or indoor planting.

3. The method according to Claim 1, **characterized in that**, wherein the carbon dioxide exists in a gaseous state;
The carbon dioxide exists in a gas mixture;
The carbon dioxide in the gas mixture may be produced by combustion;
The carbon dioxide in the gas mixture may be produced by biological metabolism;
The carbon dioxide in the gas mixture may be produced by the oxidation of CH₄ via biological or abiotic ways;
The gas mixture may be diluted with air before entering the culture container;
The gas mixture may be diluted with N₂ before entering the culture container;
The gas mixture contains no substances potentially toxic to the peat moss.

4. The method according to Claim 1, **characterized in that**:
The culture container is highly light-transmissive, with at least a top side thereof being light-transmissive;
The culture container is equipped with temperature and humidity sensors;
The humidity sensor used in the culture container is configured to transmit the humidity data within the container to an information terminal via wireless or wired means;
A lighting system is provided above the culture container;
The culture container features a sealable opening for replenishing water and nutrients;
The culture container is equipped with ports connecting to the outside, including an air inlet and an air outlet;
The culture container contains a certain water-absorbing and water-retaining substrate, or a sufficient amount of water, for planting peat moss or other plants.

5. The method according to Claim 4, **characterized in that**:
The substrate within the culture container is peat moss or a composite substrate containing peat moss;
The substrate within the culture container is peat or a composite substrate containing peat;
The substrate within the culture container is a composite substrate containing polyglutamic acid;
The substrate within the culture container is a composite culture medium containing agar;
The air inlet may lead directly into the planted peat moss clusters or into the aqueous culture solution.

6. The method according to Claim 4, **characterized in that**:
The light-transmissive surface of the culture container exhibits high transmittance for visible light in the wavelength of 350-750nm;
The lighting system can provide a visible light source with a wavelength of 300-760nm required for plant growth, or an LED light source suitable for the growth of peat moss;
The lighting system comprises a device for adjusting light intensity and photoperiod;
The humidity for peat moss planting must be controlled at 75%-100%;
The temperature for peat moss planting must be controlled at 15-28°C;
The light intensity for peat moss planting must be controlled at 1,000-10,000lux;
The photoperiod for peat moss planting must be controlled at 8-14 hours of light per day.

7. The method according to Claim 1, **characterized in that** the peat moss planting requires periodic replenishment of mineral elements, including nitrate nitrogen, phosphorus, potassium, magnesium, iron, and calcium, wherein:
In the mineral supplement mixture for the peat moss, the concentration of nitrate nitrogen is 20mM or below;
In the mineral supplement mixture for the peat moss, the concentration of phosphorus is 5mM or below;
In the mineral supplement mixture for the peat moss, the concentration of potassium is 25mM or below;
In the mineral supplement mixture for the peat moss, the concentration of magnesium is 5mM or below;
In the mineral supplement mixture for the peat moss, the concentration of calcium is 10mM or below;
In the mineral supplement mixture for the peat moss, the concentration of iron is 0.5mM or below.

8. A system for collecting and fixing carbon dioxide based on the carbon sequestration plant peat moss, used for the method according to any one of Claims 1-7, **characterized in that** it comprises: an air inlet control device (1), a plant culture device (2), and an air outlet control device (3), wherein:
The plant culture device (2) is connected to the air inlet control device (1) via an air inlet;
The air inlet control device (1) is equipped with an air inlet control port which controls the entry of external air into the air inlet control device (1), as well as a vent connecting with the plant culture device (2);
The vent of the air inlet control device (1) is equipped with a valve that controls the entry of gas into the plant culture device (2);
The plant culture device (2) is in connection with the air outlet control device (3);
The air outlet control device (3) is equipped with an exhaust control valve (6); the exhaust control valve (6) is designed for manual or automatic control; wherein the automatically controlled exhaust control valve (6) detects the carbon dioxide concentration within the air outlet control device (3) and controls the opening of the exhaust control valve (6) by determining whether the gas meets the discharge standard;
Where the exhaust control valve (6) does not connect with the outside for venting, the exhaust control valve (6) is connected to the air inlet control device (1);
The system for collecting and fixing carbon dioxide features a gas circulation pump (9) which is designed to drive gas flow into the plant culture device (2) and installed at a position that can drive gas flow within the culture container.

9. The system for collecting and fixing carbon dioxide according to Claim 8, **characterized in that**:
The air inlet control port is equipped with a first vent valve (4);
and/or,
The gas circulation pump (9) is positioned between the air inlet control device (1) and the plant culture device (2);
and/or,
The air inlet control device (1) comprises a first real-time carbon dioxide concentration sensor (7) which is configured to transmit carbon dioxide concentration data of the air inlet control device (1) to an information terminal via wired or wireless means;
and/or,
The air outlet control device (3) comprises a second carbon dioxide concentration detector (8) which is configured to transmit carbon dioxide concentration data of the air outlet control device (3) to an information terminal via wired or wireless means;
and/or,
The plant culture device (2) is provided with a lid (11) at the top, which is provided with a nutrient feeder (10);
and/or,
The gas outlet of the plant culture device (2) is provided with a second vent valve (5);
and/or,
The system for collecting and fixing carbon dioxide also comprises a visible light source.

10. Use of the system for collecting and fixing carbon dioxide according to Claim 8 in planting peat moss and collecting methane.
